(19) Europäisches Patentamt
European Patent Office
Office européen des brevets

(11) Veröffentlichungsnummer: **0 158 137**
**B1**

(12) # EUROPÄISCHE PATENTSCHRIFT

(45) Veröffentlichungstag der Patentschrift: **10.01.90**

(51) Int. Cl.⁴: **C 07 D 239/26**, C 09 K 19/00, C 07 D 239/30

(21) Anmeldenummer: **85102775.5**

(22) Anmeldetag: **12.03.85**

(54) **Pyrimidine.**

(30) Priorität: **29.03.84 DE 3411571**

(43) Veröffentlichungstag der Anmeldung: **16.10.85 Patentblatt 85/42**

(45) Bekanntmachung des Hinweises auf die Patenterteilung: **10.01.90 Patentblatt 90/2**

(84) Benannte Vertragsstaaten: **CH DE FR GB LI**

(56) Entgegenhaltungen: **DE-A- 2 547 737**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Krause, Joachim, Dr., Samuel-Morse-Strasse 14, D-6110 Dieburg (DE)**
Erfinder: **Wächtler, Andreas, Dr., Goethestrasse 34, D-6103 Griesheim (DE)**
Erfinder: **Hittich, Reinhard, Dr., Am Kirchberg 11, D-6101 Modautal 1 (DE)**
Erfinder: **Scheuble, Bernhard, Dr., Am Grenzweg 18, D-6146 Alsbach (DE)**
Erfinder: **Weber, Georg, Wilhelm-Leuschner-Strasse 38, D-6106 Erzhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft Pyrimidine der Formel I

$$R^1\text{–}A^1\text{–}Z^1\text{–}A^2\text{–}R^2 \qquad I$$

worin

$R^1$ und $R^2$ jeweils eine Alkylgruppe mit 1–15 C-Atomen, worin auch eine oder zwei nicht benachbarte $CH_2$-Gruppen durch –O–, –CO–, –CO–O–, –O–CO–, –S–, –SO– oder $–SO_2–$ ersetzt sein können,

$A^1$ –A–, $–A^3–A–$ oder $–A–A^3–$,

A 4-Fluorpyrimidin-2,5-diyl

$A^2$ und $A^3$ jeweils einen oder zwei gleiche oder verschiedene Reste ausgewählt aus der Gruppe bestehend aus 1,4-Phenylen, worin auch eine oder zwei CH-Gruppen durch N-Atome ersetzt sein können, 1,4-Cyclohexylen, worin auch eine oder zwei nicht benachbarte $CH_2$-Gruppen durch O-Atome ersetzt sein können, 1,3-Dithian-2,5-diyl oder 1,4-Bicyclo(2,2,2)octylen, und

$Z^1$ –CO–O–, –O–CO–, $–CH_2–CH_2–$, $–OCH_2–$, $–CH_2O–$ oder eine Einfachbindung

bedeutet.

Der Einfachheit halber bedeuten im folgenden Cy eine 1,4-Cyclohexylengruppe, Dio eine 1,3-Dioxan-2,5-diylgruppe, Dit eine 1,3-Dithian-2,5-diylgruppe, Bi eine 1,4-Bicyclo(2,2,2)-octylengruppe, Phe eine 1,4-phenylengruppe, Pyr eine 4-Fluor-Pyrimidin-2,5-diylgruppe und Pyn eine Pyridazin-3, 6-diylgruppe.

Die Verbindungen der Formel I können als Komponenten flüssigkristalliner Phasen verwendet werden, insbesondere für Displays, die auf dem Prinzip der verdrillten Zelle (TN-Displays), dem Guest-Host-Effekt, dem Effekt der Deformation aufgerichteter Phasen oder dem Effekt der dynamischen Streuung beruhen.

Strukturell ähnliche Verbindungen ($R^1$ = CN, $A^1$ = 4-chlor-pyrimidin-2,5-diyl, $Z^1$ = Einfachbindung, $A^2$ = 1,4-Phenylen, $R^2$ = $C_{3-9}$-Alkyl) sind bereits aus DE-OS-2 547 737 bekannt.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, dass die Verbindungen der Formel I als Komponenten flüssigkristalliner Phasen vorzüglich geeignet sind. Insbesondere sind mit ihrer Hilfe stabile flüssigkristalline Phasen mit steilen Transmissionskennlinien und relativ niedriger Viskosität herstellbar, die gleichzeitig keine oder eine nur geringe Tendenz zur Bildung smektischer Phasen aufweisen. Sie ermöglichen damit die Entwicklung hochmultiplexbarer Mischungen. Theoretische Rechnungen und experimentelle Messung haben gezeigt, daß die Transmissionskennlinie einer verdrillten Zelle (TN-Display) um so steiler wird, je kleiner das Verhältnis der Franckschen elastischen Konstanten für die Biegung und die Spreizung $K_3/K_1$ der flüssigkristallinen Phase ist. Frühere Untersuchungen ergaben teilweise sehr kleine Verhältnisse $K_3/K_1$ in Mischungen mit Alkyl-Alkoxy-Phenylpyrimidinen. Dialkyl-Phenylpyrimidine weisen noch bessere $K_3/K_1$-Werte auf, gleichzeitig treten bei diesen Verbindungen jedoch smektische Phasen auf. Überraschenderweise zeigen nun die erfindungsgemäßen Fluorpyrimidine kleine Verhältnisse $K_3/K_1$ und gleichzeitig eine signifikant erhöhte Nematogenität.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemisch eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zur Herstellung von Verbindungen der Formel I nach Anspruch 1, dadurch gekennzeichnet, daß man zur Herstellung von 4-Fluorpyrimidin-Derivaten der Formel I (worin A 4-Fluorpyrimidin-2,5-diyl bedeutet) ein entsprechendes 4-Chlor-, 4-Brom- oder 4-Jodpyrimidin-Derivat mit einem fluorierenden Mittel behandelt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Die Verbindungen der Formel I umfassen dementsprechend bevorzugte Verbindungen der Teilformeln Ia–Ic (ohne Brückenglied $Z^1$) und Id–If (mit Brückenglied $Z^1$):

$R^1$–Pyr–$A^2$–$R^2$ Ia

$R^1$–$A^3$–Pyr–$A^2$–$R^2$ Ib

$R^1$–Pyr–$A^3$–$A^2$–$R^2$ Ic

$R^1$–Pyr–$Z^1$–$A^2$–$R^2$ Id

$R^1$–$A^3$–Pyr–$Z^1$–$A^2$–$R^2$ Ie

$R^1$–Pyr–$A^3$–$Z^1$–$A^2$–$R^2$ If

Teilformel Ia umfaßt Verbindungen der Teilformeln Iaa bis Iaj:

$R^1$–Pyr–Phe–$R^2$ Iaa

$R^1$–Pyr–Cy–$R^2$ Iab

$R^1$–Pyr–Phe–Phe–$R^2$ Iac

| | |
|---|---|
| $R^1$–Pyr–Cy–Cy–$R^2$ | Iad |
| $R^1$–Pyr–Cy–Dio–$R^2$ | Iae |
| $R^1$–Pyr–Cy–Dit–$R^2$ | Iaf |
| $R^1$–Pyr–Cy–Phe–$R^2$ | Iag |
| $R^1$–Pyr–Phe–Cy–$R^2$ | Iah |
| $R^1$–Pyr–Phe–Dio–$R^2$ | Iai |
| $R^1$–Pyr–Phe–Dit–$R^2$ | Iaj |

Darunter sind diejenigen der Teilformeln Iaa–Iaf und Iah–Iaj besonders bevorzugt.

Teilformel Ib umfaßt Verbindungen der Teilformeln Iba bis Ibr:

| | |
|---|---|
| $R^1$–Cy–Pyr–Cy–$R^2$ | Iba |
| $R^1$–Cy–Pyr–Phe–$R^2$ | Ibb |
| $R^1$–Phe–Pyr–Phe–$R^2$ | Ibc |
| $R^1$–Cy–Pyr–Cy–Cy–$R^2$ | Ibd |
| $R^1$–Cy–Pyr–Phe–Phe–$R^2$ | Ibe |
| $R^1$–Cy–Pyr–Cy–Dio–$R^2$ | Ibf |
| $R^1$–Cy–Pyr–Cy–Dit–$R^2$ | Ibg |
| $R^1$–Cy–Pyr–Cy–Phe–$R^2$ | Ibh |
| $R^1$–Cy–Pyr–Phe–Cy–$R^2$ | Ibi |
| $R^1$–Cy–Pyr–Phe–Dio–$R^2$ | Ibj |
| $R^1$–Cy–Pyr–Phe–Dit–$R^2$ | Ibk |
| $R^1$–Phe–Pyr–Cy–Cy–$R^2$ | Ibl |
| $R^1$–Phe–Pyr–Phe–Cy–$R^2$ | Ibm |
| $R^1$–Phe–Pyr–Phe–Dio–$R^2$ | Ibn |
| $R^1$–Phe–Pyr–Phe–Dit–$R^2$ | Ibo |
| $R^1$–Phe–Pyr–Cy–Dio–$R^2$ | Ibp |
| $R^1$–Phe–Pyr–Cy–Dit–$R^2$ | Ibq |
| $R^1$–Phe–Pyr–Cy–Phe–$R^2$ | Ibr |

Darunter sind diejenigen der Teilformeln Ibb, Ibc, Ibe, Ibi und Ibj besonders bevorzugt.

Teilformel Ic umfaßt Verbindungen der Teilformeln Ica bis Icn:

| | |
|---|---|
| $R^1$–Pyr–Cy–Cy–$R^2$ | Ica |
| $R^1$–Pyr–Phe–Cy–$R^2$ | Icb |
| $R^1$–Pyr–Phe–Phe–$R^2$ | Icc |
| $R^1$–Pyr–Cy–Dio–$R^2$ | Icd |
| $R^1$–Pyr–Cy–Phe–$R^2$ | Ice |
| $R^1$–Pyr–Phe–Dio–$R^2$ | Icf |
| $R^1$–Pyr–Phe–Dit–$R^2$ | Icg |
| $R^1$–Pyr–Cy–Cy–Cy–$R^2$ | Ich |
| $R^1$–Pyr–Phe–Phe–Cy–$R^2$ | Ici |
| $R^1$–Pyr–Cy–Cy–Dio–$R^2$ | Icj |
| $R^1$–Pyr–Phe–Cy–Cy–$R^2$ | Ick |
| $R^1$–Pyr–Phe–Phe–Dio–$R^2$ | Icl |
| $R^1$–Pyr–Phe–Phe–Dit–$R^2$ | Icm |
| $R^1$–Pyr–Phe–Cy–Dio–$R^2$ | Icn |

Darunter sind diejenigen der Teilformeln Ica–Icd, Icf, Icg, Ich, Ici, Icj und Ick besonders bevorzugt.

Teilformel Id umfaßt die Verbindungen der Teilformeln Ida bis Ide:

| | |
|---|---|
| $R^1$–Pyr–CO–O–$A^2$–$R^2$ | Ida |
| $R^1$–Pyr–O–CO–$A^2$–$R^2$ | Idb |
| $R^1$–Pyr–$CH_2CH_2$–$A^2$–$R^2$ | Idc |
| $R^1$–Pyr–$CH_2O$–$A^2$–$R^2$ | Idd |
| $R^1$–Pyr–$OCH_2$–$A^2$–$R^2$ | Ide |

Teilformel Ie umfaßt Verbindungen der Teilformeln Iae bis Iee:

| | |
|---|---|
| $R^1$–$A^3$–Pyr–CO–O–$A^2$–$R^2$ | Iea |
| $R^1$–$A^3$–Pyr–O–CO–$A^2$–$R^2$ | Ieb |
| $R^1$–$A^3$–Pyr–$CH_2CH_2$–$A^2$–$R^2$ | Iec |
| $R^1$–$A^3$–Pyr–$CH_2O$–$A^2$–$R^2$ | Ied |
| $R^1$–$A^3$–Pyr–$OCH_2$–$A^2$–$R^2$ | Iec |

Teilformel If umfaßt Verbindungen der Teilformeln Ifa bis Ife:

| | |
|---|---|
| $R^1$–Pyr–$A^3$–CO–O–$A^2$–$R^2$ | Ifa |
| $R^1$–Pyr–$A^3$–O–CO–$A^2$–$R^2$ | Ifb |
| $R^1$–Pyr–$A^3$–$CH_2CH_2$–$A^2$–$R^2$ | Ifc |
| $R^1$–Pyr–$A^3$–$CH_2O$–$A^2$–$R^2$ | Ifd |
| $R^1$–Pyr–$A^3$–$OCH_2$–$A^2$–$R^2$ | Ife |

Darunter sind diejenigen der Teilformeln Ifa bis Ifc, insbesondere Ifc, besonders bevorzugt.

In den Teilformeln Ida–Ide, Iea–Iee und Ifa–Ife haben $A^2$ und $A^3$ die bei den Unterformeln zu Ia, Ib und Ic angegebenen bevorzugten Bedeutungen.

In den Verbindungen der vor- und nachstehenden Formeln bedeuten $R^1$ und $R^2$ vorzugsweise Alkyl, Alkoxy oder eine andere Oxaalkylgruppe. Besonders bevorzugt sind weiterhin Verbindungen der vor- und nachstehenden Formeln, worin einer Reste $R^1$ und $R^2$ Alkyl und der andere Rest Alkoxy bedeutet. Weiterhin bevorzugt sind Verbindungen der vor- und nachstehenden Formeln, worin beide Reste $R^1$ und $R^2$ jeweils unabhängig voneinander n-Alkyl bedeuten.

A ist 4-Fluorpyrimidin-2,5-diyl. $A^2$ und $A^3$ sind vorzugsweise jeweils ein oder zwei gleiche oder verschiedene Reste, ausgewählt aus der Gruppe bestehend aus Cy, Dio, Dit und Phe, insbesondere aus Cy und Phe. $Z^1$ ist bevorzugt eine Einfachbindung, in zweiter Linie bevorzugt –CO–O–, –O–CO– oder –$CH_2CH_2$–.

Falls $R^1$ und/oder $R^2$ Alkylreste bedeuten, in denen auch eine («Alkoxy» bzw. «Oxaalkyl») oder zwei («Alkoxyalkoxy» bzw. «Dioxaalkyl») nicht benachbarte $CH_2$-Gruppen durch O-Atome ersetzt sein können, so können sie geradkettig oder verzweigt sein. Vorzugsweise sind sie geradkettig, haben 2, 3, 4, 5, 6, 7, 8, 9, 10, 11 oder 12 C-Atome und bedeuten demnach bevorzugt Ethyl, Propyl, Butyl, Pentyl, Hexyl, Heptyl, Octyl, Nonyl, Decyl, Undecyl, Dodecyl, Ethoxy, Propoxy, Butoxy, Pentoxy, Hexoxy, Heptoxy, Octoxy, Nonoxy, Decoxy, Undecoxy, Dodecoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl), 2-, 3-, 4-, 5- oder 6-Oxaheptyl, ferner Methyl, Methoxy, Tridecyl, Tridecoxy, Tetradecyl, Tetradecoxy, Pentadecyl, Pentadecoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Octyloxy, 2-Oxa-3-methylbutyl, 3-Oxa-4-methylpentyl.

Unter den Verbindungen der vor- und nachstehenden Formeln I sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln Ia bis I23:

| | |
|---|---|
| Alkyl–Pyr–Phe–Alkyl | I1 |
| Alkyl–Pyr–Cy–Alkyl | I2 |
| Alkyl–Pyr–Phe–Phe–Alkyl | I3 |
| Alkyl–Pyr–Cy–Cy–Alkyl | I4 |
| Alkyl–Pyr–Phe–Cy–Alkyl | I5 |
| Alkyl–Cy–Pyr–Cy–Alkyl | I6 |
| Alkyl–Cy–Pyr–Phe–Alkyl | I7 |
| Alkyl–Phe–Pyr–Phe–Alkyl | I8 |
| Alkyl–Cy–Pyr–Phe–Cy–Alkyl | I9 |
| Alkyl–Phe–Pyr–Cy–Cy–Alkyl | I10 |
| Alkyl–Pyr–Cy–Dio–Alkyl | I11 |
| Alkyl–Pyr–Phe–Dio–Alkyl | I12 |
| Alkyl–Pyr–Phe–Dit–Alkyl | I13 |
| Alkyl–Pyr–Phe–Cy–Cy–Alkyl | I14 |
| Alkyl–Pyr–$CH_2CH_2$–Cy–Alkyl | I15 |
| Alkyl–Pyr–$CH_2CH_2$–Phe–Alkyl | I16 |
| Alkyl–Phe–Pyr–$CH_2CH_2$–Cy–Alkyl | I17 |
| Alkyl–Phe–Pyr–$CH_2CH_2$–Phe–Alkyl | I18 |
| Alkyl–Pyr–Phe–$CH_2CH_2$–Cy–Alkyl | I19 |
| Alkyl–Pyr–Phe–$CH_2CH_2$–Phe–Alkyl | I20 |
| Alkyl–Pyr–Phe–$CH_2CH_2$–Cy–Cy–Alkyl | I21 |
| Alkyl–Pyr–Phe–$OCH_2$–Cy–Alkyl | I22 |
| Alkyl–Pyr–Phe–$OCH_2$–Phe–Alkyl | I23 |

Ebenfalls bevorzugt sind die den Formeln I1 bis I23 entsprechenden Alkyl-Alkoxy-Verbindungen, worin eine der Alkylgruppen in I1 bis I23 durch eine Alkoxygruppe ersetzt ist. Alkyl bzw. Alkoxy bedeutet jeweils eine geradkettige Alkyl- bzw. Alkoxygruppe mit 2 bis 12 C-Atomen.

Unter den Verbindungen der Formel I sind diejenigen Stereoisomeren bevorzugt, in denen die gesättigten Reste $A^2$ und $A^3$ (z. B. Cy, Dio, Dit) trans-1,4-disubstituiert sind.

Diejenigen der vorstehend genannten Formeln, die eine oder mehrere der Gruppen Dio, Dit und/oder Pyr enthalten, umschließen jeweils die beiden möglichen 2,5-Stellungsisomeren.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der Fachmann kann durch Routinemethoden entsprechende Synthesemethoden aus dem Stand der Technik entnehmen (z. B. DE-OS-2 344 732, 2 450 088, 2 429 093, 2 502 904, 2 636 684, 2 701 591 und 2 752 975 betreffend Verbindungen mit 1,4-Cyclohexylen- und 1,4-Phenylen-Gruppen; DE-PS-2 641 724 betreffend Verbindungen mit Pyrimidin-2,5-diyl-Gruppen; DE-OS-3 238 350 betreffend Verbindungen mit Pyridazin-2,5-diyl-Gruppen; JP-OS-58-43 961 betreffend Verbindungen mit Pyrazin-2,5-diyl-Gruppen; DE-OS-2 944 905 und 3 227 916 betreffend Verbindungen mit 1,3-Dioxan-2,5-diyl-Gruppen; DD-160 061 betreffend Verbindungen mit 1,3-Dithian-2,5-diyl-Gruppen; US-4 261 652 und 4 219 256 betreffend Verbindungen mit 1,4-Bicyclo-(2,2,2)-octylen-Gruppen; DE-3 201 721 betreffend Verbindungen mit $-CH_2CH_2-$Brückengliedern; DE-OS-2 139 628, DE-OS-2 535 046 und DE-OS-2 800 553 betreffend Verbindungen mit $Z^1$ = –CO–O– oder –O–CO– und DE-OS-3 001 661, DE-OS-3 040 632 und DE-OS-3 149 139 betreffend Verbindungen mit $Z^1$ = $-CH_2O-$ bzw. $-OCH_2-$).

Weiterhin können jedoch auch entsprechende intramolekulare Kondensationen zur Synthese von Verbindungen der Formel I durchgeführt werden (z. B. Kondensation von 1,4-Diketonen mit Hydrazin (z. B. DE-OS-3 238 350) oder Umsetzung eines Butadienderivates z. B. mit Acetylendicarbonsäurederivaten (z. B. JP-OS-58-144 327; JP-OS-58-146 543)).

Die Ausgangsstoffe sind bekannt oder können nach analogen Methoden wie die bekannten Verbindungen erhalten werden. Der Fachmann kann durch Routinemethoden entsprechende Ausgangsstoffe und/oder Methoden zu deren Synthese aus dem Stand der Technik entnehmen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

4-Fluorpyrimidin-Derivate der Formel I können hergestellt werden, indem man ein entsprechende 4-Chlor-, 4-Brom- oder 4-Jod-pyrimidin-Derivat mit einem fluorierenden Mittel behandelt. Die als Ausgangsmaterialien eingesetzten Halogenpyrimidine sind teilweise bekannt oder können nach analogen Methoden wie die bekannten Verbindungen erhalten werden, z. B. durch Umsetzung entsprechender Hydroxypyrimidine mit Phosphoroxychlorid [D. J. Brown, The Pyrimidines in A. Weissgerber (Hrsg.): The Chemistry of Heterocyclic Compounds, Interscience Publishers New York – London 1962, Seite 162–216]. Die Hydroxypyrimidine ihrerseits sind durch Kondensation und Ringschluß entsprechender Amidine mit 2-substituierten 2-Alkoxy-acrylsäureestern erhältlich [A. Boller, M. Cereghetti und H. Scherrer, Z. Naturforsch. 33b, 433–438 (1978)].

Als fluorierende Mittel eignen sich in erster Linie Silberfluorid [Wempen und Fox, J. Med. Chem. 6, 688 (1963)], Kaliumfluorid [Finger und Starr, J. Amer. Chem. Soc. 81, 2674 (1959)], Schwefeltetrafluorid oder Diethylaminschwefeltrifluorid [J. Org. Chem. 40 (5), 574–8 (1975)], die unter Reaktionsbedingungen eingesetzt werden, die für die genannten Umsetzungen bekannt und geeignet sind.

Die erfindungsgemäßen flüssigkristallinen Phasen bestehen aus 2 bis 15, vorzugsweise 3 bis 12 Komponenten, darunter mindestens einer Verbindung der Formel I. Die anderen Bestandteile wer-

den vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexyl-cyclohexane, Cyclohexylnaphthaline, 1,4-Bis-cyclohexylbenzole, 4,4'-Biscyclohexylbiphenyle, Phenyl- oder Cyclohexylpyrimidine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexyl-ethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

$$R'\text{–}L\text{–}G\text{–}E\text{–}R'' \qquad II$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | |
|---|---|
| G –CH=CH– | –N(O)=N– |
| –CH=CY– | –CH=N(O)– |
| –C≡C– | $-CH_2CH_2-$ |
| –CO–O– | $-CH_2-O-$ |
| –CO–S– | $-CH_2-S-$ |
| –CH=N– | –COO–Phe–COO– |

oder eine C–C-Einfachbindung, Y Halogen, vorzugsweise Chlor, oder –CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Phasen enthalten etwa 0,1 bis 99, vorzugsweise 10 bis 95%, einer oder mehrerer Verbindungen der Formel I.

Weiterhin bevorzugt sind erfindungsgemäße Dielektrika enthaltend 0,1 bis 40, vorzugsweise 0,5 bis 30%, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxy-benzoat,
Tetrabutylammonium-tetraphenylboranat
oder Komplexsalze von Kronenethern (vgl. z. B. I. Haller et al., Mol. Cryst. Liq. Cryst. Band 24, Seiten 249–258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS-2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. Vor- und nachstehend bedeuten Prozentangaben Gewichtsprozent; alle Temperaturen sind in Grad Celsius angegeben. «Übliche Aufarbeitung» bedeutet: man gibt Wasser hinzu, extrahiert mit Methylenchlorid, trennt ab, trocknet die organische Phase, dampft ein und reinigt das Produkt durch Kristallisation und/oder Chromatographie.

Beispiel 1

43 g 2-(p-Propylphenyl)-4-chlor-5-heptylpyrimidin [hergestellt aus p-Propylbenzamidinhydrochlorid und 2-Ethoxymethylennonancarbonsäureethylester durch basische Kondensation und Ringschluß (EtOH/NaOEt) und nachfolgender Umsetzung mit $POCl_3$] und 27 g Schwefeltetrafluorid werden in einem Schüttelautoklav 5 Stunden auf 120° erhitzt. Das Reaktionsprodukt wird in Methylenchlorid aufgenommen und wie üblich aufgearbeitet. Man erhält 2-(p-Propylphenyl)-4-fluor-5-heptylpyrimidin.

Analog werden hergestellt:

2-(p-Propylphenyl)-4-fluor-5-propylpyrimidin
2-(p-Propylphenyl)-4-fluor-5-butylpyrimidin
2-(p-Propylphenyl)-4-fluor-5-pentylpyrimidin
2-(p-Propylphenyl)-4-fluor-5-hexylpyrimidin
2-(p-Propylphenyl)-4-fluor-5-octylpyrimidin
2-(p-Propylphenyl)-4-fluor-5-nonylpyrimidin
2-(p-Propylphenyl)-4-fluor-5-decylpyrimidin
2-(p-Propylphenyl)-4-fluor-5-undecylpyrimidin
2-(p-Propylphenyl)-4-fluor-5-dodecylpyrimidin

2-(p-Butylphenyl)-4-fluor-5-propylpyrimidin
2-(p-Butylphenyl)-4-fluor-5-butylpyrimidin
2-(p-Butylphenyl)-4-fluor-5-pentylpyrimidin
2-(p-Butylphenyl)-4-fluor-5-hexylpyrimidin
2-(p-Butylphenyl)-4-fluor-5-octylpyrimidin
2-(p-Butylphenyl)-4-fluor-5-nonylpyrimidin
2-(p-Butylphenyl)-4-fluor-5-decylpyrimidin
2-(p-Butylphenyl)-4-fluor-5-undecylpyrimidin
2-(p-Butylphenyl)-4-fluor-5-dodecylpyrimidin

2-(p-Pentylphenyl)-4-fluor-5-propylpyrimidin
2-(p-Pentylphenyl)-4-fluor-5-butylpyrimidin

2-(p-Pentylphenyl)-4-fluor-5-pentylpyrimidin
2-(p-Pentylphenyl)-4-fluor-5-hexylpyrimidin
2-(p-Pentylphenyl)-4-fluor-5-octylpyrimidin
2-(p-Pentylphenyl)-4-fluor-5-nonylpyrimidin
2-(p-Pentylphenyl)-4-fluor-5-decylpyrimidin
2-(p-Pentylphenyl)-4-fluor-5-undecylpyrimidin
2-(p-Pentylphenyl)-4-fluor-5-dodecylpyrimidin

2-(p-Heptylphenyl)-4-fluor-5-propylpyrimidin
2-(p-Heptylphenyl)-4-fluor-5-butylpyrimidin
2-(p-Heptylphenyl)-4-fluor-5-pentylpyrimidin
2-(p-Heptylphenyl)-4-fluor-5-hexylpyrimidin
2-(p-Heptylphenyl)-4-fluor-5-octylpyrimidin
2-(p-Heptylphenyl)-4-fluor-5-nonylpyrimidin
2-(p-Heptylphenyl)-4-fluor-5-decylpyrimidin
2-(p-Heptylphenyl)-4-fluor-5-undecylpyrimidin
2-(p-Heptylphenyl)-4-fluor-5-dodecylpyrimidin

2-(p-Methoxyphenyl)-4-fluor-5-propylpyrimidin
2-(p-Methoxyphenyl)-4-fluor-5-butylpyrimidin
2-(p-Methoxyphenyl)-4-fluor-5-pentylpyrimidin
2-(p-Methoxyphenyl)-4-fluor-5-hexylpyrimidin
2-(p-Methoxyphenyl)-4-fluor-5-octylpyrimidin
2-(p-Methoxyphenyl)-4-fluor-5-nonylpyrimidin
2-(p-Methoxyphenyl)-4-fluor-5-decylpyrimidin
2-(p-Methoxyphenyl)-4-fluor-5-undecylpyrimidin
2-(p-Methoxyphenyl)-4-fluor-5-dodecylpyrimidin

2-(p-Ethoxyphenyl)-4-fluor-5-propylpyrimidin
2-(p-Ethoxyphenyl)-4-fluor-5-butylpyrimidin
2-(p-Ethoxyphenyl)-4-fluor-5-pentylpyrimidin
2-(p-Ethoxyphenyl)-4-fluor-5-hexylpyrimidin
2-(p-Ethoxyphenyl)-4-fluor-5-octylpyrimidin
2-(p-Ethoxyphenyl)-4-fluor-5-nonylpyrimidin
2-(p-Ethoxyphenyl)-4-fluor-5-decylpyrimidin
2-(p-Ethoxyphenyl)-4-fluor-5-undecylpyrimidin
2-(p-Ethoxyphenyl)-4-fluor-5-dodecylpyrimidin

2-(p-Propoxyphenyl)-4-fluor-5-propylpyrimidin
2-(p-Propoxyphenyl)-4-fluor-5-butylpyrimidin
2-(p-Propoxyphenyl)-4-fluor-5-pentylpyrimidin
2-(p-Propoxyphenyl)-4-fluor-5-hexylpyrimidin
2-(p-Propoxyphenyl)-4-fluor-5-octylpyrimidin
2-(p-Propoxyphenyl)-4-fluor-5-nonylpyrimidin
2-(p-Propoxyphenyl)-4-fluor-5-decylpyrimidin
2-(p-Propoxyphenyl)-4-fluor-5-undecylpyrimidin
2-(p-Propoxyphenyl)-4-fluor-5-dodecylpyrimidin

2-(p-Pentoxyphenyl)-4-fluor-5-propylpyrimidin
2-(p-Pentoxyphenyl)-4-fluor-5-butylpyrimidin
2-(p-Pentoxyphenyl)-4-fluor-5-pentylpyrimidin
2-(p-Pentoxyphenyl)-4-fluor-5-hexylpyrimidin
2-(p-Pentoxyphenyl)-4-fluor-5-octylpyrimidin
2-(p-Pentoxyphenyl)-4-fluor-5-nonylpyrimidin
2-(p-Pentoxyphenyl)-4-fluor-5-decylpyrimidin
2-(p-Pentoxyphenyl)-4-fluor-5-undecylpyrimidin
2-(p-Pentoxyphenyl)-4-fluor-5-dodecylpyrimidin

Beispiel 2

1,9 g 2-[2-(trans-4-Propylcyclohexyl)-ethyl]-4-chlor-5-(p-pentylphenyl)-pyrimidin werden in 150 ml Methylenchlorid gelöst und bei −50° zu einer Lösung von 2,1 ml Diethylaminoschwefeltrifluorid und 50 ml Methylenchlorid getropft. Nach 6 Stunden wird das Gemisch auf Raumtemperatur ewärmen gelassen und anschließend noch 8 Stunden bei 70° gerührt. Nach üblicher Aufarbeitung erhält man 2-[2-(trans-4-Propylcyclohexyl)-ethyl]-4-fluor-5-(p-pentylphenyl)-pyrimidin.

Analog werden hergestellt:

2-[2-(trans-4-Propylcyclohexyl)-ethyl]-4-fluor-5-(p-propylphenyl)-pyrimidin
2-[2-(trans-4-Propylcyclohexyl)-ethyl]-4-fluor-5-(p-butylphenyl)-pyrimidin

2-[2-(trans-4-Propylcyclohexyl)-ethyl]-4-fluor-5-(p-heptylphenyl)-pyrimidin
2-[2-(trans-4-Propylcyclohexyl)-ethyl]-4-fluor-5-(p-nonylphenyl)-pyrimidin
2-[2-(trans-4-Propylcyclohexyl)-ethyl]-4-fluor-5-(p-decylphenyl)-pyrimidin

2-[2-(trans-4-Butylcyclohexyl)-ethyl]-4-fluor-5-(p-propylphenyl)-pyrimidin
2-[2-(trans-4-Butylcyclohexyl)-ethyl]-4-fluor-5-(p-butylphenyl)-pyrimidin
2-[2-(trans-4-Butylcyclohexyl)-ethyl]-4-fluor-5-(p-heptylphenyl)-pyrimidin
2-[2-(trans-4-Butylcyclohexyl)-ethyl]-4-fluor-5-(p-nonylphenyl)-pyrimidin
2-[2-(trans-4-Butylcyclohexyl)-ethyl]-4-fluor-5-(p-decylphenyl)-pyrimidin

2-[2-(trans-4-Pentylcyclohexyl)-ethyl]-4-fluor-5-(p-propylphenyl)-pyrimidin
2-[2-(trans-4-Pentylcyclohexyl)-ethyl]-4-fluor-5-(p-butylphenyl)-pyrimidin
2-[2-(trans-4-Pentylcyclohexyl)-ethyl]-4-fluor-5-(p-heptylphenyl)-pyrimidin
2-[2-(trans-4-Pentylcyclohexyl)-ethyl]-4-fluor-5-(p-nonylphenyl)-pyrimidin
2-[2-(trans-4-Pentylcyclohexyl)-ethyl]-4-fluor-5-(p-decylphenyl)-pyrimidin

1-(trans-4-Propylcyclohexyl)-2-p-(4-fluor-2-propylpyrimidin-5-yl)-phenyl-ethan
1-(trans-4-Propylcyclohexyl)-2-p-(4-fluor-2-butylpyrimidin-5-yl)-phenyl-ethan
1-(trans-4-Propylcyclohexyl)-2-p-(4-fluor-2-pentylpyrimidin-5-yl)-phenyl-ethan
1-(trans-4-Propylcyclohexyl)-2-p-(4-fluor-2-heptylpyrimidin-5-yl)-phenyl-ethan
1-(trans-4-Propylcyclohexyl)-2-p-(4-fluor-2-nonylpyrimidin-5-yl)-phenyl-ethan
1-(trans-4-Propylcyclohexyl)-2-p-(4-fluor-2-decylpyrimidin-5-yl)-phenyl-ethan

1-(trans-4-Pentylcyclohexyl)-2-p-(4-fluor-5-propylpyrimidin-2-yl)-phenyl-ethan
1-(trans-4-Pentylcyclohexyl)-2-p-(4-fluor-5-butylpyrimidin-2-yl)-phenyl-ethan
1-(trans-4-Pentylcyclohexyl)-2-p-(4-fluor-5-pentylpyrimidin-2-yl)-phenyl-ethan
1-(trans-4-Pentylcyclohexyl)-2-p-(4-fluor-5-heptylpyrimidin-2-yl)-phenyl-ethan
1-(trans-4-Pentylcyclohexyl)-2-p-(4-fluor-5-nonylpyrimidin-2-yl)-phenyl-ethan
1-(trans-4-Pentylcyclohexyl)-2-p-(4-fluor-5-decylpyrimidin-2-yl)-phenyl-ethan

2-(trans-4-Propylcyclohexyl)-4-fluor-5-propyl-pyrimidin
2-(trans-4-Propylcyclohexyl)-4-fluor-5-butyl-pyrimidin
2-(trans-4-Propylcyclohexyl)-4-fluor-5-pentyl-pyrimidin
2-(trans-4-Propylcyclohexyl)-4-fluor-5-heptyl-pyrimidin
2-(trans-4-Propylcyclohexyl)-4-fluor-5-nonyl-pyrimidin
2-(trans-4-Propylcyclohexyl)-4-fluor-5-decyl-pyrimidin

2-(trans-4-Pentylcyclohexyl)-4-fluor-5-propyl-pyrimidin
2-(trans-4-Pentylcyclohexyl)-4-fluor-5-butyl-pyrimidin
2-(trans-4-Pentylcyclohexyl)-4-fluor-5-pentyl-pyrimidin
2-(trans-4-Pentylcyclohexyl)-4-fluor-5-heptyl-pyrimidin
2-(trans-4-Pentylcyclohexyl)-4-fluor-5-nonyl-pyrimidin
2-(trans-4-Pentylcyclohexyl)-4-fluor-5-decyl-pyrimidin

2-(trans-4-Heptylcyclohexyl)-4-fluor-5-propyl-pyrimidin
2-(trans-4-Heptylcyclohexyl)-4-fluor-5-butyl-pyrimidin
2-(trans-4-Heptylcyclohexyl)-4-fluor-5-pentyl-pyrimidin
2-(trans-4-Heptylcyclohexyl)-4-fluor-5-heptyl-pyrimidin
2-(trans-4-Heptylcyclohexyl)-4-fluor-5-nonyl-pyrimidin
2-(trans-4-Heptylcyclohexyl)-4-fluor-5-decyl-pyrimidin

2-(trans-4-Propylcyclohexyl)-4-fluor-5-(p-propylphenyl)-pyrimidin
2-(trans-4-Propylcyclohexyl)-4-fluor-5-(p-butylphenyl)-pyrimidin
2-(trans-4-Propylcyclohexyl)-4-fluor-5-(p-pentylphenyl)-pyrimidin
2-(trans-4-Propylcyclohexyl)-4-fluor-5-(p-heptylphenyl)-pyrimidin
2-(trans-4-Propylcyclohexyl)-4-fluor-5-(p-nonylphenyl)-pyrimidin
2-(trans-4-Propylcyclohexyl)-4-fluor-5-(p-decylphenyl)-pyrimidin

2-(trans-4-Pentylcyclohexyl)-4-fluor-5-(p-propylphenyl)-pyrimidin
2-(trans-4-Pentylcyclohexyl)-4-fluor-5-(p-butylphenyl)-pyrimidin
2-(trans-4-Pentylcyclohexyl)-4-fluor-5-(p-pentylphenyl)-pyrimidin
2-(trans-4-Pentylcyclohexyl)-4-fluor-5-(p-heptylphenyl)-pyrimidin
2-(trans-4-Pentylcyclohexyl)-4-fluor-5-(p-nonylphenyl)-pyrimidin
2-(trans-4-Pentylcyclohexyl)-4-fluor-5-(p-decylphenyl)-pyrimidin

2-(p-Pentylphenyl)-4-fluor-5-(p-propylphenyl)-pyrimidin
2-(p-Pentylphenyl)-4-fluor-5-(p-butylphenyl)-pyrimidin
2-(p-Pentylphenyl)-4-fluor-5-(p-pentylphenyl)-pyrimidin
2-(p-Pentylphenyl)-4-fluor-5-(p-heptylphenyl)-pyrimidin
2-(p-Pentylphenyl)-4-fluor-5-(p-nonylphenyl)-pyrimidin
2-(p-Pentylphenyl)-4-fluor-5-(p-decylphenyl)-pyrimidin

2-(4′-Pentylbiphenyl-4-yl)-4-fluor-5-propyl-pyrimidin
2-(4′-Pentylbiphenyl-4-yl)-4-fluor-5-butyl-pyrimidin
2-(4′-Pentylbiphenyl-4-yl)-4-fluor-5-pentyl-pyrimidin
2-(4′-Pentylbiphenyl-4-yl)-4-fluor-5-heptyl-pyrimidin
2-(4′-Pentylbiphenyl-4-yl)-4-fluor-5-nonyl-pyrimidin
2-(4′-Pentylbiphenyl-4-yl)-4-fluor-5-decyl-pyrimidin

2-(4′-Propylbiphenyl-4-yl)-4-fluor-5-propyl-pyrimidin
2-(4′-Propylbiphenyl-4-yl)-4-fluor-5-butyl-pyrimidin
2-(4′-Propylbiphenyl-4-yl)-4-fluor-5-pentyl-pyrimidin
2-(4′-Propylbiphenyl-4-yl)-4-fluor-5-heptyl-pyrimidin
2-(4′-Propylbiphenyl-4-yl)-4-fluor-5-nonyl-pyrimidin
2-(4′-Propylbiphenyl-4-yl)-4-fluor-5-decyl-pyrimidin

2-(trans-4-Propylcyclohexyl)-4-fluor-5-(p-propylphenyl)-pyrimidin
2-(trans-4-Propylcyclohexyl)-4-fluor-5-(p-butylphenyl)-pyrimidin
2-(trans-4-Propylcyclohexyl)-4-fluor-5-(p-pentylphenyl)-pyrimidin
2-(trans-4-Propylcyclohexyl)-4-fluor-5-(p-heptylphenyl)-pyrimidin
2-(trans-4-Propylcyclohexyl)-4-fluor-5-(p-nonylphenyl)-pyrimidin
2-(trans-4-Propylcyclohexyl)-4-fluor-5-(p-decylphenyl)-pyrimidin

2-(trans-4-Butylcyclohexyl)-4-fluor-5-(p-propylphenyl)-pyrimidin
2-(trans-4-Butylcyclohexyl)-4-fluor-5-(p-butylphenyl)-pyrimidin
2-(trans-4-Butylcyclohexyl)-4-fluor-5-(p-pentylphenyl)-pyrimidin
2-(trans-4-Butylcyclohexyl)-4-fluor-5-(p-heptylphenyl)-pyrimidin
2-(trans-4-Butylcyclohexyl)-4-fluor-5-(p-nonylphenyl)-pyrimidin
2-(trans-4-Butylcyclohexyl)-4-fluor-5-(p-decylphenyl)-pyrimidin

Die folgenden Beispiele betreffen erfindungsgemäße flüssigkristalline Phasen:

Beispiel A

Eine flüssigkristalline Phase aus

4,4% 4-Ethyl-4'-cyanbiphenyl,
3,8% 4-Propyl-4'-cyanbiphenyl,
4,3% 4-Butyl-4'-cyanbiphenyl,
3,5% 4-Cyan-4'-(trans-4-pentylcyclohexyl)-biphenyl,
1,0% 4-Ethyl-4'-(trans-4-propylcyclohexyl)-biphenyl,
7,5% 4-Ethyl-2'-fluor-4'-(trans-4-pentylcyclohexyl)-biphenyl,
4,0% p-trans-4-Propylcyclohexyl-benzoesäure-(p-propylphenylester),
3,0% p-trans-4-Pentylcyclohexyl-benzoesäure-(p-propylphenylester),
10,6% trans-4-Pentylcyclohexancarbonsäure-(p-pentylphenylester),
6,4% trans-4-Propylcyclohexancarbonsäure-(p-ethoxyphenylester),
2,5% 4,4'-Bis-(trans-4-propylcyclohexyl)-biphenyl,
2,0% 4-(trans-4-Pentylcyclohexyl)-4'-(trans-4-propylcyclohexyl)-biphenyl,
0,5% 2-[2-(trans-4-Propylcyclohexyl)-ethyl]-4-fluor-5-(p-pentylphenyl)-pyrimidin,
8,5% trans-1-p-Ethylphenyl-4-propylcyclohexan,
5,0% trans-1-p-Ethoxyphenyl-4-propylcyclohexan
5,5% 2-p-Hexyloxyphenyl-5-hexylpyrimidin,
5,5% 2-p-Heptyloxyphenyl-5-hexylpyrimidin,
5,5% 2-p-Octyloxyphenyl-5-hexylpyrimidin,
5,5% 2-p-Nonyloxyphenyl-5-hexylpyrimidin,
5,5% 2-p-Decyloxyphenyl-5-hexylpyrimidin und
5,5% 2-p-Dodecyloxyphenyl-5-hexylpyrimidin,

hat einen Schmelzpunkt von −11°, einen Klärpunkt von 71° und ist gut geeignet als hoch multiplexierbares Dielektrikum.

Beispiel B

Eine flüssigkristalline Phase aus

6,6% 4-Ethyl-4'-cyanbiphenyl,
5,4% 4-Propyl-4'-cyanbiphenyl,
4,6% 4-Butyl-4'-cyanbiphenyl,
11,4% trans-1-p-Ethylphenyl-4-propylcyclohexan,
6,6% trans-1-p-Ethoxyphenyl-4-propylcyclohexan,
4,6% 4-Cyan-4'-(trans-4-pentylcyclohexyl)-biphenyl,
1,4% 4-Ethyl-4'-(trans-4-propylcyclohexyl)-biphenyl,
10,0% 4-Ethyl-2'-fluor-4'-(trans-4-pentylcyclohexyl)-biphenyl,
5,4% p-trans-4-Propylcyclohexyl-benzoesäure-(p-propylphenylester),
4,0% p-trans-4-Pentylcyclohexyl-benzoesäure-(p-propylphenylester),
12,2% 2-(p-Propylphenyl)-4-fluor-5-heptylpyrimidin,
17,8% 2-(p-Propylphenyl)-4-fluor-5-nonylpyrimidin,
1,6% 2-p-Hexyloxyphenyl-5-hexylpyrimidin,
1,7% 2-p-Heptyloxyphenyl-5-hexylpyrimidin,
1,7% 2-p-Octyloxyphenyl-5-hexylpyrimidin,
1,7% 2-p-Nonyloxyphenyl-5-hexylpyrimidin,
1,7% 2-p-Decyloxyphenyl-5-hexylpyrimidin und
1,6% 2-p-Dodecyloxyphenyl-5-hexylpyrimidin

ist ein hoch multiplexierbares Dielektrikum.

**Patentansprüche**

1. Pyrimidine der Formel I

$$R^1-A^1-Z^1-A^2-R^2 \qquad I$$

worin

$R^1$ und $R^2$ jeweils eine Alkylgruppe mit 1–15 C-Atomen, worin auch eine oder zwei nicht benachbarte $CH_2$-Gruppen durch –O–, –CO–, –CO–O–, –O–CO–, –S–, –SO– oder $-SO_2-$ ersetzt sein können,

$A^1$ –A–, $-A^3-A-$ oder $-A-A^3-$,

A 4-Fluorpyrimidin-2,5-diyl (Pyr),

$A^2$ und $A^3$ jeweils einen oder zwei gleiche oder verschiedene Reste ausgewählt aus der Gruppe bestehend aus 1,4-Phenylen (Phe), worin auch eine oder zwei CH-Gruppen durch N-Atome ersetzt sein können, 1,4-Cyclohexylen (Cy), worin auch eine oder zwei nicht benachbarte $CH_2$-Gruppen durch O-Atome ersetzt sein können, 1,3-Dithian-2,5-diyl (Dit) oder 1,4-Bicyclo (2,2,2)octylen (Bi), und

$Z^1$ –CO–O–, –O–CO–, $-CH_2-CH_2-$, $-OCH_2-$, $-CH_2O-$ oder eine Einfachbindung

bedeutet.

2. Pyrimidine nach Anspruch 1, dadurch gekennzeichnet, daß $A^2$ und $A^3$ jeweils trans-1,4-Cyclohexylen oder 1,4-Phenylen bedeuten.

3. Pyrimidine nach Anspruch 1 oder 2, dadurch gekennzeichnet, daß $Z^1$ $-CH_2CH_2-$ oder eine Einfachbindung ist.

4. Pyrimidine nach Anspruch 1, 2 oder 3, dadurch gekennzeichnet, daß $R^1$ und $R^2$ geradkettiges Alkyl oder Alkoxy mit bis zu 12 C-Atomen bedeuten.

5. Pyrimidine nach Anspruch 1, gekennzeichnet durch die Formeln I1 bis I23:

| | |
|---|---|
| Alkyl–Pyr–Phe–Alkyl | I1 |
| Alkyl–Pyr–Cy–Alkyl | I2 |
| Alkyl–Pyr–Phe–Phe–Alkyl | I3 |
| Alkyl–Pyr–Cy–Cy–Alkyl | I4 |
| Alkyl–Pyr–Phe–Cy–Alkyl | I5 |
| Alkyl–Cy–Pyr–Cy–Alkyl | I6 |
| Alkyl–Cy–Pyr–Phe–Alkyl | I7 |
| Alkyl–Phe–Pyr–Phe–Alkyl | I8 |
| Alkyl–Cy–Pyr–Phe–Cy–Alkyl | I9 |
| Alkyl–Phe–Pyr–Cy–Cy–Alkyl | I10 |
| Alkyl–Pyr–Cy–Dio–Alkyl | I11 |
| Alkyl–Pyr–Phe–Dio–Alkyl | I12 |
| Alkyl–Pyr–Phe–Dit–Alkyl | I13 |
| Alkyl–Pyr–Phe–Cy–Cy–Alkyl | I14 |
| Alkyl–Pyr–$CH_2CH_2$–Cy–Alkyl | I15 |
| Alkyl–Pyr–$CH_2CH_2$–Phe–Alkyl | I16 |
| Alkyl–Phe–Pyr–$CH_2CH_2$–Cy–Alkyl | I17 |
| Alkyl–Phe–Pyr–$CH_2CH_2$–Phe–Alkyl | I18 |
| Alkyl–Pyr–Phe–$CH_2CH_2$–Cy–Alkyl | I19 |
| Alkyl–Pyr–Phe–$CH_2CH_2$–Phe–Alkyl | I20 |
| Alkyl–Pyr–Phe–$CH_2CH_2$–Cy–Cy–Alkyl | I21 |
| Alkyl–Pyr–Phe–$OCH_2$–Cy–Alkyl | I22 |
| Alkyl–Pyr–Phe–$OCH_2$–Phe–Alkyl | I23 |

sowie die den Formeln I1 bis I23 entsprechenden Alkyl-Alkoxy-Verbindungen, worin eine der Alkylgruppen in I1 bis I23 durch eine Alkoxygruppe ersetzt ist, wobei Alkyl bzw. Alkoxy jeweils eine geradkettige Alkyl- bzw. Alkoxygruppe mit 2 bis 12 C-Atomen bedeutet.

6. Verwendung der Verbindungen der Formel I nach Anspruch 1 als Komponenten flüssigkristalliner Phasen.

7. Flüssigkristalline Phase mit mindestens zwei flüssigkristallinen Komponenten, dadurch gekennzeichnet, daß mindestens eine Komponente eine Verbindung der Formel I nach Anspruch 1 ist.

8. Flüssigkristallananzeigeelement, dadurch gekennzeichnet, daß es eine Phase nach Anspruch 7 enthält.

9. Elektrooptisches Anzeigeelement nach Anspruch 8, dadurch gekennzeichnet, daß es als Dielektrikum eine Phase nach Anspruch 7 enthält.

## Claims

1. Pyrimidines of the formula I

$$R^1-A^1-Z^1-A^2-R^2 \quad I$$

wherein

$R^1$ and $R^2$ are each an alkyl group with 1–15 C atoms, it also being possible for one or two non-adjacent $CH_2$ groups to be replaced by –O–, –CO–, –CO–O–, –O–CO–, –S–, –SO– or $-SO_2-$,

$A^1$ is –A–, $-A^3-A-$ or $-A-A^3-$,

A is 4-fluoropyrimidine-2,5-diyl (Pyr)

$A^2$ and $A^3$ are each one or two identical or different radicals selected from the group consisting of 1,4-phenylene (Phe), it also being possible for one or two CH groups to be replaced by N atoms, 1,4-cyclohexylene (Cy), it also being possible for one or two non-adjacent $CH_2$ groups to be replaced by 0 atoms, 1,3-dithiane-2,5-diyl (Dit) or 1,4-bicyclo(2,2,2)octylen (Bi), and

$Z^1$ is –CO–O–, –O–CO–, $-CH_2-CH_2-$, $-OCH_2-$, $-CH_2O-$ or a single bond.

2. Pyrimidines according to Claim 1 characterized in, that $A^2$ and $A^3$ are each trans-1,4-cyclohexylene or 1,4-phenylene.

3. Pyrimidines according to Claim 1 or 2 characterized in, that $Z^1$ is $-CH_2CH_2-$ or a single bond.

4. Pyrimidines according to Claim 1, 2 or 3 characterized in, that $R^1$ and $R^2$ denote normal alkyl or alcoxy with up to 12 C atoms.

5. Pyrimidines according to Claim 1 characterized by the formulae I1 to I23:

| | |
|---|---|
| Alkyl–Pyr–Phe–Alkyl | I1 |
| Alkyl–Pyr–Cy–Alkyl | I2 |
| Alkyl–Pyr–Phe–Phe–Alkyl | I3 |
| Alkyl–Pyr–Cy–Cy–Alkyl | I4 |
| Alkyl–Pyr–Phe–Cy–Alkyl | I5 |
| Alkyl–Cy–Pyr–Cy–Alkyl | I6 |
| Alkyl–Cy–Pyr–Phe–Alkyl | I7 |
| Alkyl–Phe–Pyr–Phe–Alkyl | I8 |
| Alkyl–Cy–Pyr–Phe–Cy–Alkyl | I9 |
| Alkyl–Phe–Pyr–Cy–Cy–Alkyl | I10 |
| Alkyl–Pyr–Cy–Dio–Alkyl | I11 |
| Alkyl–Pyr–Phe–Dio–Alkyl | I12 |
| Alkyl–Pyr–Phe–Dit–Alkyl | I13 |
| Alkyl–Pyr–Phe–Cy–Cy–Alkyl | I14 |
| Alkyl–Pyr–$CH_2CH_2$–Cy–Alkyl | I15 |
| Alkyl–Pyr–$CH_2CH_2$–Phe–Alkyl | I16 |
| Alkyl–Phe–Pyr–$CH_2CH_2$–Cy–Alkyl | I17 |
| Alkyl–Phe–Pyr–$CH_2CH_2$–Phe–Alkyl | I18 |
| Alkyl–Pyr–Phe–$CH_2CH_2$–Cy–Alkyl | I19 |
| Alkyl–Pyr–Phe–$CH_2CH_2$–Phe–Alkyl | I20 |
| Alkyl–Pyr–Phe–$CH_2CH_2$–Cy–Cy–Alkyl | I21 |
| Alkyl–Pyr–Phe–$OCH_2$–Cy–Alkyl | I22 |
| Alkyl–Pyr–Phe–$OCH_2$–Phe–Alkyl | I23 |

and the alkyl-alcoxy-compounds corresponding to the formulae I1 to I23 wherein one of the alkyl groups of the formulae I1 to I23 is replaced by a alcoxy group, wherein «Alkyl» or «Alcoxy» respectively each denote a normal alkyl or alcoxy group respectively with 2 to 12 C atoms.

6. Use of the compounds of the formula I according to Claim 1 as components of liquid crystal phases.

7. Liquid crystal phase with at least two liquid crystal components, characterised in that at least one component is a compound of the formula I according to Claim 1.

8. Liquid crystal display element, characterised in that it contains a phase according to Claim 7.

9. Electrooptical display element according to Claim 8, characterised in that it contains a phase according to Claim 7 as the dielectric.

## Revendications

1. Pyrimidines de formule I

$$R^1-A^1-Z^1-A^2-R^2 \quad I$$

dans laquelle

$R^1$ et $R^2$ représentent chacun un groupe alkyle en C1–C15 dans lequel un ou deux groupes $CH_2$ non voisins peuvent être remplacés par –O–, –CO–, –CO–O–, –O–CO–, –S–, –SO– ou $-SO_2-$,

$A^1$ représente –A–, $-A^3-A-$ ou $-A-A^3-$,

A représente un groupe 4-fluoropyrimidine-2,5-diyle (Pyr),

$A^2$ et $A^3$ représentent chacun un ou deux radicaux identiques ou différents choisis parmi les groupes 1,4-phénylène (Phe) dans lesquels également un ou deux groupes $CH_2$ peuvent être remplacés par des atomes d'azote, 1,4-cyclohexylène (Cy) dans lesquels également un ou deux groupes $CH_2$ non voisins peuvent être remplacés par des atomes d'oxygène, 1,3-dithianne-2,5-diyle (Dit) ou 1,4-bicyclo (2,2,2)octylène (Bi) et

$Z^1$ représente –CO–O–, –O–CO–, $-CH_2-CH_2-$, $-OCH_2-$, $-CH_2O-$ ou une liaison simple.

2. Pyrimidines selon la revendication 1, caractérisées en ce que $A^2$ et $A^3$ représentent chacun un groupe trans-1,4-cyclohexylène ou 1,4-phénylène.

3. Pyrimidines selon la revendication 1 ou 2, caractérisées en ce que $Z^1$ représente $-CH_2CH_2-$ ou une liaison simple.

4. Pyrimidines selon la revendication 1, 2 ou 3, caractérisées en ce que $R^1$ et $R^2$ représentent des groupes alkyle ou alcoxy à chaîne droite contenant jusqu'à 12 atomes de carbone.

5. Pyrimidines selon la revendication 1, caractérisées par les formules I1 à I23:

| | |
|---|---|
| Alkyl–Pyr–Phe–Alkyle | I1 |
| Alkyl–Pyr–Cy–Alkyle | I2 |
| Alkyl–Pyr–Phe–Phe–Alkyle | I3 |
| Alkyl–Pyr–Cy–Cy–Alkyle | I4 |
| Alkyl–Pyr–Phe–Cy–Alkyle | I5 |
| Alkyl–Cy–Pyr–Cy–Alkyle | I6 |
| Alkyl–Cy–Pyr–Phe–Alkyle | I7 |
| Alkyl–Phe–Pyr–Phe–Alkyle | I8 |
| Alkyl–Cy–Pyr–Phe–Cy–Alkyle | I9 |
| Alkyl–Phe–Pyr–Cy–Cy–Alkyle | I10 |
| Alkyl–Pyr–Cy–Dio–Alkyle | I11 |
| Alkyl–Pyr–Phe–Dio–Alkyle | I12 |
| Alkyl–Pyr–Phe–Dit–Alkyle | I13 |
| Alkyl–Pyr–Phe–Cy–Cy–Alkyle | I14 |
| Alkyl–Pyr–$CH_2CH_2$–Cy–Alkyle | I15 |
| Alkyl–Pyr–$CH_2CH_2$–Phe–Alkyle | I16 |
| Alkyl–Phe–Pyr–$CH_2CH_2$–Cy–Alkyle | I17 |
| Alkyl–Phe–Pyr–$CH_2CH_2$–Phe–Alkyle | I18 |
| Alkyl–Pyr–Phe–$CH_2CH_2$–Cy–Alkyle | I19 |
| Alkyl–Pyr–Phe–$CH_2CH_2$–Phe–Alkyle | I20 |
| Alkyl–Pyr–Phe–$CH_2CH_2$–Cy–Cy–Alkyle | I21 |
| Alkyl–Pyr–Phe–$OCH_2$–Cy–Alkyle | I22 |
| Alkyl–Pyr–Phe–$OCH_2$–Phe–Alkyle | I23 |

ainsi que les composés alkyl-alcoxylés répondant aux formules I1 à I23, dans lesquelles l'un des groupes alkyle est remplacé par un groupe alcoxy, les notations «Alkyl» et «Alcoxy» désignant respectivement des groupes alkyle et alcoxy à chaîne droite en C2–C12.

6. Utilisation des composés de formule I selon la revendication 1 en tant que composants de phases à cristaux liquides.

7. Phase à cristaux liquides à au moins deux composants à cristaux liquides, caractérisée en ce que l'un au moins des composants est un composé de formule I selon la revendication 1.

8. Elément d'affichage à cristaux liquides, caractérisé en ce qu'il contient une phase selon la revendication 7.

9. Elément d'affichage électro-optique selon la revendication 8, caractérisé en ce qu'il contient en tant que diélectrique une phase selon la revendication 7.